# EUROPEAN PATENT APPLICATION

(11) **EP 3 666 878 A1**
(43) Date of publication of application: **17.06.2020**
(21) Application number: 19201814.1
(22) Date of filing: 19.05.2014
(51) Int. Cl.: C12M 1/00, C12M 1/32, G01N 1/16

(54) **ASEPTIC SAMPLING MODULE AND MANIFOLD**

(30) Priority: 06.06.2013 US 201361832101 P; 30.01.2014 US 201461933806 P
(62) Divisional of application: 14732491.7
(71) Applicant: Bend Research, Inc., Bend, OR 97701 (US)
(72) Inventor: NEWBOLD, David, Dixon, Bend, Oregon 97701 (US)
(74) Representative: De Clercq & Partners

(57) **Abstract**

A sample can be collected from an enclosed container, such as an aseptic bioreactor or chemical environment, using the disclosed sampling modules and manifold, providing consistent or substantially consistent sampling procedures for obtaining samples of a desired quality, while reducing the risk of contamination. The samples can be directed to appropriate analytical devices for analysis.

## Description

### FIELD

The present disclosure is directed to an automatic aseptic sampling module and manifold and methods of using the same.

### BACKGROUND

Obtaining samples from containers or other systems that support biologically and/or chemically active environments can require complex and careful sampling procedures to avoid contamination of the containers or the environment itself. For example, most bioreactors require frequent sampling (e.g., one or more times a day) to monitor and control the conditions and levels of nutrients needed for cell growth. To reduce the risk of contamination within such systems, conventional sampling techniques generally require operators to perform multiple, labor-intensive steps.

In addition, samples from one aseptic source are typically directed to several analytical devices to measure various properties of the contents of the source, such as pH, dissolved oxygen, osmolality, nutrient concentrations, ammonia/ammonium, lactate/lactic acid, pCO₂, electrolytes (such as K+, Ca++, and/or Na+), amino acids, NAD/NADH, impurities, purity, phenotypes, metabolic states, cell cycle, or other properties.

### SUMMARY

In some embodiments, the aseptic sampling module and manifold disclosed herein provides consistent or substantially consistent sampling procedures for obtaining samples of a desired quality, while reducing the risk of contamination of the aseptic fluids and directing them to appropriate analytical devices. In some embodiments, samples from multiple sources may be directed to multiple analytical devices for analysis.

In one embodiment, a sample-directing manifold comprises (a) a plurality of sample inlets, wherein each sample inlet is in fluid communication with a respective sample outlet valve and a respective waste outlet valve; (b) a sample outlet path in fluid communication with each respective sample outlet valve; and (c) a waste outlet path in fluid communication with each said respective waste outlet valve.

In one embodiment, the sample-directing manifold is arranged such that each respective waste outlet valve and each respective sample outlet valve may be configured independently so that only one of the plurality of sample inlets may be in fluid communication with the sample outlet path.

In one embodiment, the sample-directing manifold further comprises a flushing fluid inlet in fluid communication with the sample outlet path.

In one embodiment, the sample-directing manifold is made from a body, the body having internal channels defining the plurality of sample inlets, the sample outlet path, and the waste outlet path. In another embodiment the body comprises channels for each of the respective sample outlet valves and each of the respective waste outlet valves.

In one embodiment, a sampling system for collecting a fluid sample from an enclosed container comprises the sample-directing manifold. In one embodiment, the sampling system further comprises an aseptic sampling valve. In one embodiment the aseptic sampling valve comprises a variable volume reservoir.

In one embodiment, the sampling system comprises a control module in communication with the sample-directing manifold and the aseptic sampling valve.

In another embodiment, a sample manifold comprises (a) a plurality of sample inlets, each sample inlet having a sample outlet valve and a waste outlet valve, (b) a sample outlet path, (c) a waste outlet path, (d) a gas inlet valve, (e) a fluid inlet valve, and (f) an outlet path isolation valve. Wherein the sample manifold can be configured so that each sample inlet can be connected to the sample outlet path through the sample outlet valve while the outlet path isolation valve is closed, while the other sample inlets are connected to the waste outlet path through the waste outlet valves, and wherein the fluid inlet can be configured to remove the sample from the sample outlet path when the outlet path isolation valve is open so as to direct the fluid to waste, and the gas inlet can be used to remove the fluid from the outlet path when the isolation valve is open, so as to prepare the outlet path for receiving another sample.

In another embodiment, a sampling system for collecting a fluid sample from an enclosed container comprises at least two modules, a control module, and a sampling module. The control module comprises (1) a source of compressed gas, (2) one or more valves for directing compressed gas to the sampling module, and (3) an optional vacuum pump. The sampling module comprises: (4) a sanitizing fluid inlet valve; (5) a gas inlet valve; (6) a sample collection valve; (7) an outlet valve; (8) a variable volume reservoir; and (9) a fluid flow path interconnecting (4) - (8).

In another embodiment, A method of collecting a fluid sample from an aseptic container, comprises: opening a sanitant inlet valve and directing sanitant through a sample outlet path, discharging said sanitant through an outlet path isolation valve; closing the sanitant inlet valve, opening a gas inlet valve to remove sanitant from the sample outlet path; opening sample inlet and a sample outlet valve to direct the sample to an analytical device.

### BRIEF DESCRIPTION OF THE DRAWINGS

FIG. 1 illustrates a schematic view of the sampling system.
FIG. 2 illustrates schematic views of another embodiment of the sampling system.
FIG. 3 illustrates a schematic view of the sample-directing manifold.
FIG. 4 illustrates a schematic view of an outlet valve for directing sample to an outlet path. The waste outlet valve is essentially of the same design.
FIG. 5 illustrates a schematic view of another embodiment of the sampling system.
FIG. 6 illustrates a schematic view of another embodiment of the sampling system.
FIG. 7 illustrates a schematic view of another embodiment of the sampling system. aseptic source 120, the at least one aseptic sampling system 130, the sample directing manifold 140, and at least one analytical device 160 are in fluid communication along a fluid flow path 105.

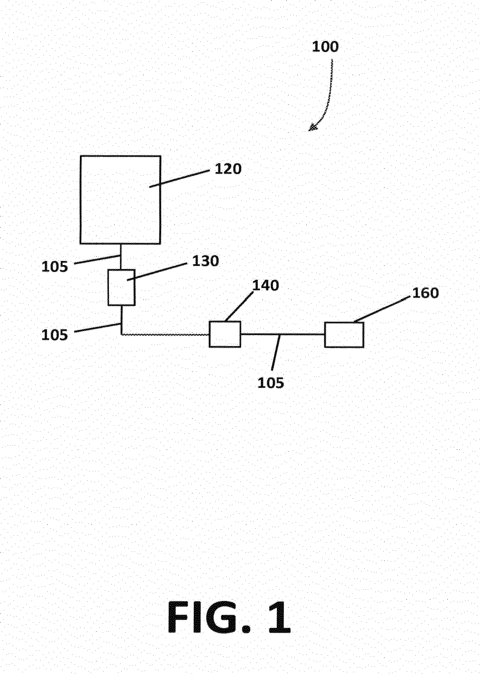

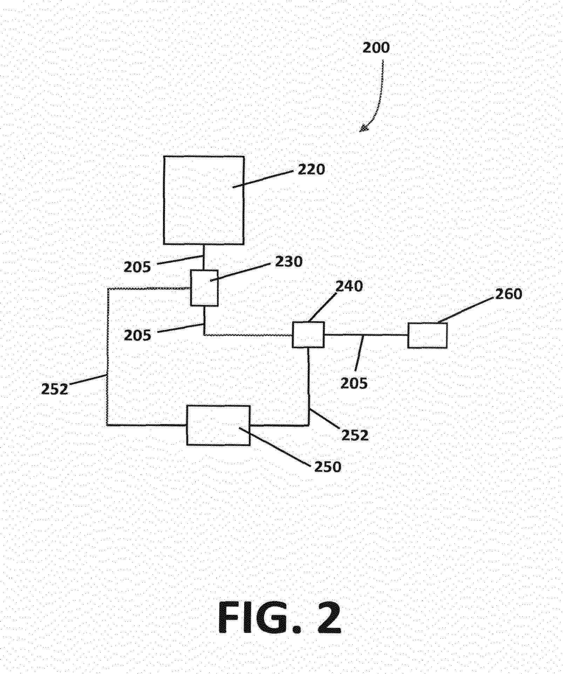

FIG. 2 illustrates another embodiment of the sampling system. FIG. 2 is similar to FIG. 1, but with the addition of a control module 250, that is in communication with the at least one aseptic sampling system 230 and a sample-directing manifold 240, through line 252.

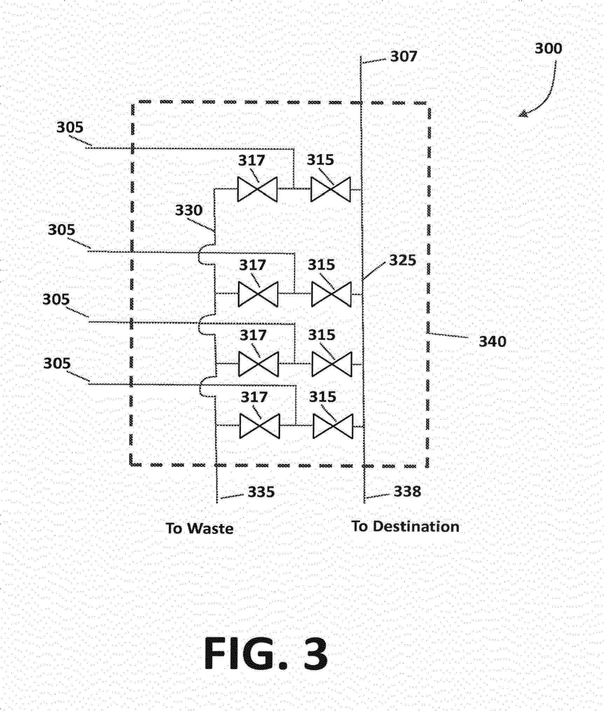

FIG. 3 illustrates a schematic of one embodiment of the sample-directing manifold 300. The sample-directing manifold 340 of FIG. 3 includes four sample inlets 305, each sample inlet 305 having a sample outlet valve 315 and a waste outlet valve 317. The sample-directing manifold also includes a flushing fluid inlet 307 that is upstream of the first sample outlet valve 315, a sample outlet path 325, and a sample outlet 338 that is downstream of the fourth sample outlet valve 315. The sample inlet 305 is located between the corresponding sample outlet valve 315 and waste outlet valve 317. The sample-directing manifold also includes a waste outlet path 330, and a waste outlet 335 that is downstream of the last waste outlet valve 317.

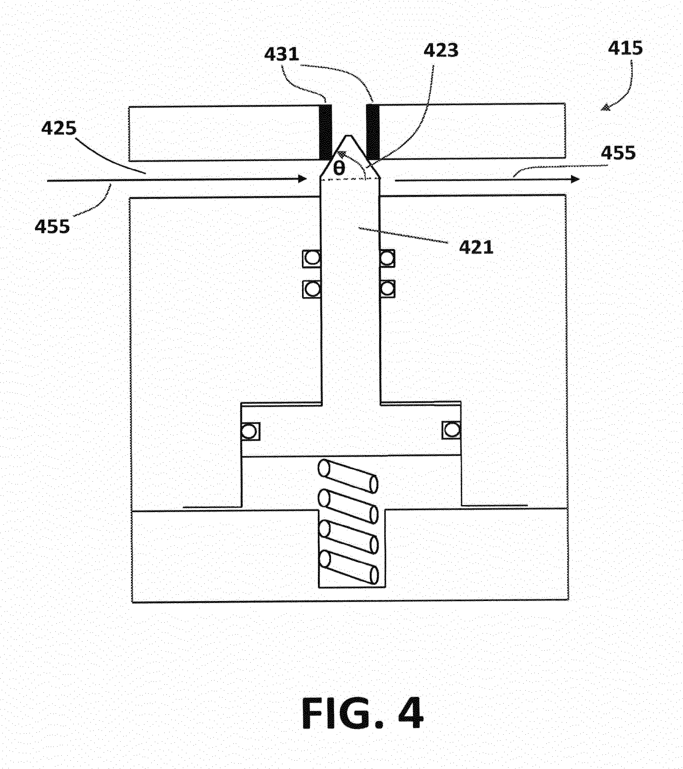

FIG. 4 illustrates an enlarged partial cross-sectional view of a sample outlet valve 415. When the sample outlet valve is in a closed position sanitant 455 can flow around the end of valve stem 421. Sealing member 423 seals the valve into seat 431. Thus, for example, as shown by arrows 455, sanitant can pass around a portion of the valve stem 421, thereby improving sanitization or sterilization of the sample outlet path 425.

The waste outlet valves 317 are of a similar design as shown in FIG. 4, and can be sanitized using a similar procedure.

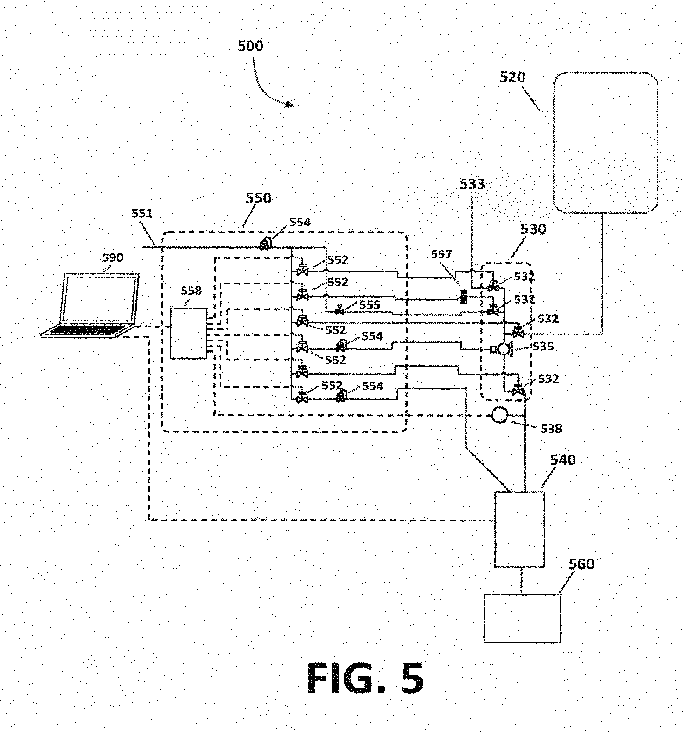

FIG. 5 shows another embodiment of the sampling system 500. FIG. 5 comprises an aseptic source 520, a control module 550, an aseptic sampling valve 530, a sample-directing manifold 540, and an analytical device 560. The control module comprises a gas source 551, a plurality of control valves 552, at least two pressure regulators 554, valve 555, and gas filter 557. The control module also includes a programmable logic controller (PLC) 558 that communicates with the components in the control module 550. A computer interface 590 is also shown for controlling the sampling system 500. The aseptic sampling valve 530 comprises valves 532 and a sample pump 535. In one embodiment, the sample pump 535 is a variable volume sample pump. In another embodiment, the sample pump 535 is a variable volume reservoir.

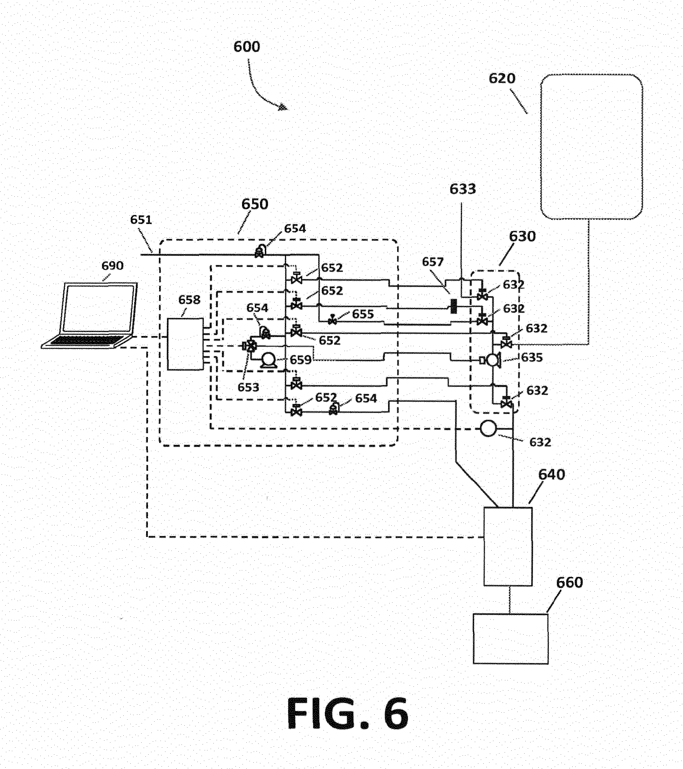

FIG. 6 shows another embodiment of the sampling system 600. FIG. 6 comprises an aseptic source 620, a control module 650, and aseptic sampling valve 630, a sample-directing manifold 640, and an analytical device 660. The control module 650 comprises a gas source 651, a plurality of control valves 652, at least three pressure regulators 654, valve 655, gas filter 657, and vacuum pump 659. The control module also includes a programmable logic controller (PLC) 658 that communicates with the components in the control module. A computer interface 690 is also shown for controlling the sampling system 600. The aseptic sampling valve 630 comprises valves 632 and a sample pump 635. In one embodiment, the sample pump 635 is a variable volume sample pump. In another embodiment, the sample pump 635 is a variable volume reservoir.

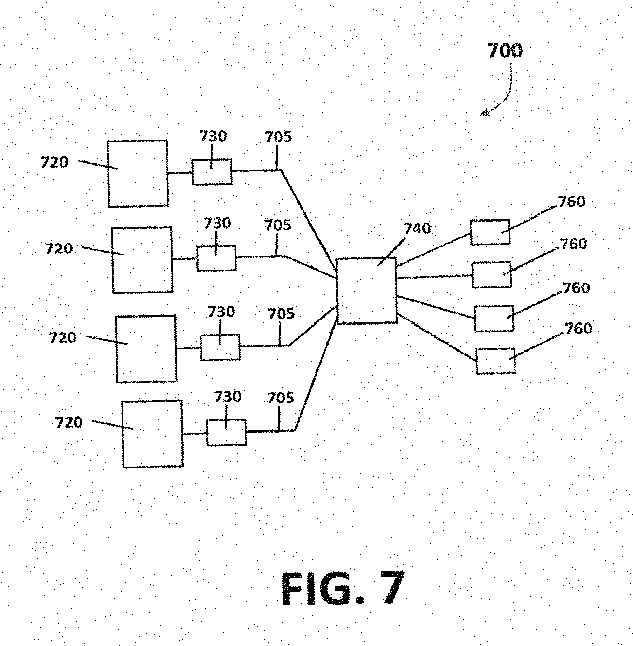

FIG. 7 illustrates another embodiment of the sampling system 700. Sampling system 700 comprises four aseptic sources 720, four aseptic sampling systems 730 in fluid communication with the corresponding aseptic sources 720. Sampling system 700 also comprises sample-directing manifold 740, in fluid communication with corresponding sample inlets 705. Sampling system 700 also comprises four analytical devices 760, in fluid communication with the sample-directing manifold 740. The sampling system 700 can be used to direct sample from each of the aseptic sources 720 to the sample-directing manifold 740 using the aseptic sampling systems 730, and direct the sample to any of the analytical devices 760, using the control module (not shown in FIG. 7).

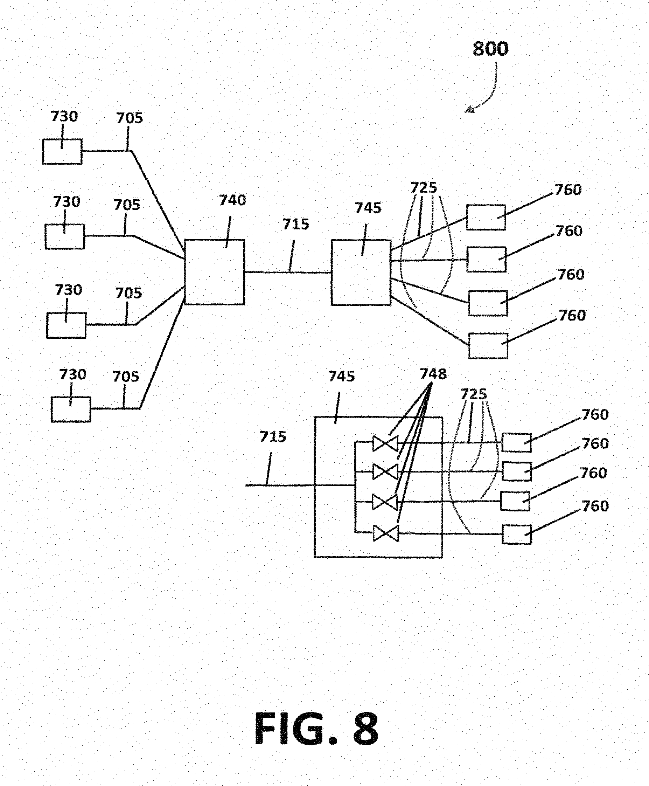

FIG. 8 illustrates another embodiment of the sampling system 800. FIG. 8 shows four aseptic sampling systems 730 in fluid communication sample-directing manifold 740 through corresponding sample inlets 705. Sample-directing manifold 740 is in communication with analytical-directing manifold 745. Analytical-directing manifold contains four sample-directing valves 748 in fluid communication through lines 725 to four analytical devices 760. In one embodiment, the sampling system 800 may be configured independently wherein the sample-directing valves 748 may be in fluid communication with only one analytical device 760. In another embodiment, the sampling system 800 may be configured independently wherein sample-directing valves 748 may be in fluid communication with two or more analytical devices 760.

In one embodiment, the sanitant is any fluid that can sanitize, disinfect, or sterilize the sampling module. The sanitant can be a liquid, a gas, or a combination thereof. Sanitants include steam, ethylene oxide, glutaraldehyde, formaldehyde, formalin, chlorine gas, hypochlorite, bromine, hypobromite, iodine, hypoiodite, bromine chloride, chlorine dioxide, ozone, hydrogen peroxide, monochloramine, dichloramine, trichloramine, quaternary ammonium salts, ethanol, 70% ethanol/water, isopropanol, 70% isopropanol/water, peroxyacetic acid, and peracetic acid. This list of possible sanitants should not be construed to indicate that all alternatives are equivalent to one another. In one embodiment, the sanitant is steam. In another embodiment, the sanitant is ethylene oxide. In another embodiment, the sanitant is glutaraldehyde. In another embodiment, the sanitant is ethanol. In another embodiment, the sanitant is a mixture of ethanol and water, such as 70% ethanol/water.

In one embodiment, the gas may be air, nitrogen, or any gas appropriate to purge the sanitant and sample from the sampling module. In one embodiment, the gas is filtered through an appropriate filter to remove contaminants that may affect the aseptic nature of the samples. In one embodiment, the gas is nitrogen. In one embodiment the gas is air.

Referring to FIG. 4, in one embodiment, the sample outlet valve 415 comprises a valve stem 421, a sealing member 423, and a seat 431. FIG. 4 illustrates that valve stem 421 tapers at the end to form sealing member 423. To provide improved sealing characteristics, in some embodiments, the tip of the valve stem (sealing member 423) can extend at an angle (θ in FIG. 4) of greater than 50 degrees from the body of the valve stem 421, in another embodiment the taper is an angle of greater than 60 degrees, in another embodiment the taper is an angle of greater than 70 degrees, and in another embodiment the taper is an angle of about 80 degrees.

In one embodiment, sealing member 423 can be formed of a material that has a lower yield strength than the material of the seat 431, into which sealing member 423 extends. In some embodiments, sealing member 423 can be made of metals, thermoplastic polymers, such as polyether-ether ketone (PEEK), polyether imide (PEI), polyphenylsulfone (PPSU), polysulfone (PSU), or combinations of these. In one embodiment, sealing member 423 is made from creep resistant metals, such as stainless steel, titanium, nickel, brass, and anodized aluminum; or high temperature thermoplastic polymers such as PEEK, or PPSU (also known as Radel®). The seat 431 can be made from a more flexible material, such as silicone rubber, polytetrafluoroethylene (PTFE, also known as Teflon®), perfluroalkoxy (PFA), ethylene tetrafluoroethylene (ETFE), high density polyethylene (HDPE), high density polypropylene (HDPP), perfluroalkoxy (PFA, also known as Viton®), and combinations thereof. In one embodiment, the valve stem 421 and the sealing member 423 are selected from metals, PEEK, PPSU, PEI, and mixtures thereof. Suitable metals include stainless steel, titanium, nickel, brass, and anodized aluminum. In one embodiment the seat is selected from PFA, PTFE, HDPE, HDPP, PFA and mixtures thereof. This list is not intended to indicate that each alternative is necessarily equivalent to the others.

In another embodiment, the sealing member 423 is selected from PEEK, PPSU, and PEI, and the seat 431 material is selected from PTFE, Teflon®, ETFE, HDPE, HDPP, PFA, due to their relatively chemically inert behavior.

In one embodiment, the sealing member and seat are arranged to form a variable sealing area when the sealing member and seat are in contact, causing the seat 431 material to deform until the stress on the materials at the seal is within the elastic modulus of the seat material, allowing a good seal even with relatively wide tolerances on the angles of the seat 431 and sealing member 423.

In another embodiment, sealing member 423 is formed of a higher yield strength material, such as a polymeric material, such as PTFE, PFA, ETFE, HDPE, HDPP, etc., while the seat 431 is formed of a more lower yield strength material, such as thermoplastic polymers (PEEK, PEI, PPSU, PSU, etc.), metals, or combinations or these materials. In this embodiment, sealing member 423 can extrude into the seat 431 to form a tight seal. In addition, in one embodiment, sealing member 423 and seat 431 can be cone shaped and may be inversely shaped such that the cone seat 431 comprises a hollow cone into which the cone shaped member 423 may fit. The sealing member 423 can have a steeper cone shape than the hollow cone seat 431, thereby allowing sealing member 423 to extrude into the seat 431 to form a positive seal.

When one or both of the sealing member 423 and seat 431 are formed of polymers, the heat up and cool down times associated with those parts can be faster than the times associated with other materials, such as steel or other metals.

In some embodiments, the sealing member and valve stem can be formed of the same polymeric material, which can further improve operation by reducing complexities of manufacturing and permitting the sealing member and valve stem component to be more compact.

In one embodiment, the sample-directing manifold comprises 2 module inlets and associated sample outlet valves and waste outlet valves. In another embodiment, the sample-directing manifold comprises 3 module inlets and associated sample outlet valves and waste outlet valves. In yet another embodiment, the sample-directing manifold comprises 4 module inlets and associated sample outlet valves and waste outlet valves. In still another embodiment, at least two sample-directing manifolds are combined to one or more analytical devices or other destinations.

In one embodiment, the analytical device may be a pH meter, a dissolved oxygen probe, an osmometer, high-performance liquid chromatograph (HPLC), a conductivity meter, a gas chromatograph, a mass spectrometer, ion chromatography, dielectric spectroscopy, microscopy, quantitative visualization tools, focused beam reflectance measurements (FBRM), particle vision and measurement (PVM) devices, a turbidity meter, reduction-oxidation probes, a flow cytometer, Raman/NIR spectroscopy, automated hemocytometer, electro-rotation, electrophoresis, dielectrophoresis, fluorescent activated cell sorting (FACS), or other analytical instruments designed to measure the properties of the aseptic fluid.

In one embodiment the sample-directing manifold comprises, consists of, or essentially consists of, a plurality of sample inlets, wherein each sample inlet is in fluid communication with a respective sample outlet valve and a respective waste outlet valve, a sample outlet path in fluid communication with each said respective sample outlet valve, and a waste outlet path in fluid communication with each said respective waste outlet valve. In certain embodiments the respective waste outlet valve and each of the respective sample outlet valves may be configured independently so that only one of said plurality of sample inlets may be in fluid communication with said sample outlet path. In other embodiments, the sample-directing manifold as disclosed immediately above further comprise a flushing fluid inlet in fluid communication with said sample outlet path. In yet other embodiments the sample-directing manifold the above embodiments are made from a body having internal channels defining said plurality of sample inlets, said sample outlet path, and said waste outlet path. In yet other embodiments, the above embodiments may comprise the body having channels for each of said respective sample outlet valves and each of said respective waste outlet valves.

In another embodiment the sampling system for collecting a fluid sample from an enclosed container comprises the sample-directing manifold of any one of the previous disclosed embodiments. In other embodiments the sampling system disclosed immediately above further comprises an aseptic sampling valve. In yet other embodiments the aseptic sampling valve in the preceding embodiments comprises a variable volume reservoir.

The sampling systems described above also may further comprise an control module in communication with said sample-directing manifold and said aseptic sampling valve.

In one embodiment the sample manifold comprises a plurality of sample inlets, each sample inlet having a sample outlet valve and a waste outlet valve, a sample outlet path, a waste outlet path, a gas inlet valve, a fluid inlet valve, and an outlet path isolation valve, wherein said sample manifold can be configured so that each sample inlet can be connected to said sample outlet path through said sample outlet valve while said outlet path isolation valve is closed, while the other sample inlets are connected to said waste outlet path through said waste outlet valves, and wherein said fluid inlet can be configured to remove said sample from said sample outlet path when said outlet path isolation valve is open so as to direct said fluid to waste, and said gas inlet can be used to remove said fluid from said outlet path when said isolation valve is open, so as to prepare said outlet path for receiving another sample.

In another embodiment the sampling system for collecting a fluid sample from an enclosed container comprises at least two modules, a control module, and a sampling module, wherein said control module comprises a source of compressed gas, one or more valves for directing compressed gas to the sampling module, and an optional vacuum pump; and the sampling module comprises a sanitizing fluid inlet valve, a gas inlet valve, a sample collection valve, an outlet valve, a variable volume reservoir, and a fluid flow path interconnecting the sanitizing fluid inlet valve, the gas inlet valve, the sample collection valve and the variable volume reservoir.

Also disclosed are certain embodiments of a method of collecting a fluid sample from an aseptic container, comprising opening a sanitizing fluid inlet valve and directing sanitizing fluid through a sample outlet path, discharging said sanitizing fluid through an outlet path isolation valve, closing the sanitizing fluid inlet valve, opening a gas inlet valve to remove sanitant from the sample outlet path, and opening a sample inlet and a sample outlet valve to direct the sample to an analytical device.

In view of the many possible embodiments to which the principles of the disclosed invention may be applied, it should be recognized that the illustrated embodiments are only preferred examples of the invention and should not be taken as limiting the scope of the invention. Rather, the scope of the invention is defined by the following claims. We therefore claim as our invention all that comes within the scope and spirit of these claims.

## Claims

1. A sample-directing manifold comprising:
(a) a plurality of sample inlets, wherein each sample inlet is in fluid communication with a respective sample outlet valve and a respective waste outlet valve;
(b) a sample outlet path in fluid communication with each said respective sample outlet valve; and
(c) a waste outlet path in fluid communication with each said respective waste outlet valve.

2. The sample-directing manifold of claim 1, wherein one or more of the respective waste outlet valves and one or more of the respective sample outlet valves may be configured independently so that only one of said plurality of sample inlets may be in fluid communication with said sample outlet path.

3. The sample-directing manifold of claims 1 and 2, further comprising a flushing fluid inlet in fluid communication with said sample outlet path.

4. The sample-directing manifold of any of the previous claims made from a body, said body having internal channels defining said plurality of sample inlets, said sample outlet path, and said waste outlet path.

5. The sample-directing manifold of claim 4, wherein said body comprises channels for each of said respective sample outlet valves and each of said respective waste outlet valves.

6. A sampling system for collecting a fluid sample from an enclosed container comprising the sample-directing manifold of any one of the previous claims.

7. The sampling system of claim 6, further comprising an aseptic sampling valve.

8. The sampling system of claim 7, wherein said aseptic sampling valve comprises a variable volume reservoir.

9. The sampling system of any of claims 6 to 8, further comprising a control module in communication with said sample-directing manifold and said aseptic sampling valve.

10. A sample manifold comprising:
(a) a plurality of sample inlets, one or more of the sample inlet having a sample outlet valves and a waste outlet valves,
(b) a sample outlet path,
(c) a waste outlet path,
(d) a gas inlet valve,
(e) a fluid inlet valve, and
(f) an outlet path isolation valve;
wherein said sample manifold can be configured so that each sample inlet can be connected to said sample outlet path through said sample outlet valve while said outlet path isolation valve is closed, while the other sample inlets are connected to said waste outlet path through said waste outlet valves, and
wherein said fluid inlet can be configured to remove said sample from said sample outlet path when said outlet path isolation valve is open so as to direct said fluid to waste, and said gas inlet can be used to remove said fluid from said outlet path when said isolation valve is open, so as to prepare said outlet path for receiving another sample.

11. A sampling system for collecting a fluid sample from an enclosed container comprising at least two modules, a control module, and a sampling module;
(A) wherein said control module comprises:
(1) a source of compressed gas,
(2) one or more valves for directing compressed gas to the sampling module, and
(3) an optional vacuum pump; and
(B) said sampling module comprises:
(4) a sanitizing fluid inlet valve;
(5) a gas inlet valve;
(6) a sample collection valve;
(7) an outlet valve;
(8) a variable volume reservoir; and
(9) a fluid flow path interconnecting (4) - (8).

12. A method of collecting a fluid sample from an aseptic container, comprising:
opening a sanitizing fluid inlet valve and directing sanitizing fluid through a sample outlet path, discharging said sanitizing fluid through an outlet path isolation valve; and
closing the sanitizing fluid inlet valve, opening a gas inlet valve to remove sanitant from the sample outlet path; opening a sample inlet and a sample outlet valve to direct the sample to an analytical device.
